# EUROPEAN PATENT SPECIFICATION

(11) **EP 0 474 868 B1**
(45) Date of publication and mention of the grant of the patent: **13.11.1996**
(21) Application number: 90907471.8
(22) Date of filing: 29.05.1990
(51) Int. Cl.: C12N 7/04, C12P 21/00, A61K 39/21

(54) **METHOD OF PRODUCING CORE STRUCTURE OF RETRO VIRUS AND PARTICLE PRODUCED THEREBY**
VERFAHREN ZUR HERSTELLUNG VON KERNSTRUKTUREN VON RETROVIREN UND DADURCH HERGESTELLTE PARTIKEL
PROCEDE DE PRODUCTION DE STRUCTURES DE NOYAU DE RETROVIRUS ET PARTICULES AINSI PRODUITES

(30) Priority: 30.05.1989 JP 136862/89
(43) Date of publication of application: 18.03.1992
(73) Proprietor: NIPPON ZEON CO., LTD., Chiyoda-ku Tokyo 100 (JP)
(72) Inventor: SHIBUTA, Hiroshi, Tokyo 182 (JP); SHIODA, Tatsuo, San Francisco, CA 94122 (US)
(74) Representative: Stuart, Ian Alexander
(86) International application number: JP9000689
(87) International publication number: WO9015133

(56) References cited:
- WO-A-88/03563
- WO-A-88/09670
- J. Virol., Vol. 63, No. 3 (1989) GOWDA S D, et al "Expression and Processing of human immunodeficiency virus type 1 gag and pol genes by cells infected with a recombinant vaccinia virus" P. 1451-1454.
- Virology, Vol. 166, No. 2 (1988), FLEXNER C, et al "Characterization of human immunodeficiency virus gag/pol gene products expressed by recombinant vaccinia viruses" p. 339-343.
- Virology, Vol. 158, No. 1 (1987) MADISEN L, et al "Expression of the human immunodeficiency virus gag gene in insert cells" p. 248-250.
- VIROLOGY, vol. 175, no. 1, March 1990, SHIODA, T. & SHIBUTA. H.: pp. 139-148

## Description

### [FIELD OF THE INVENTION]

The present invention relates to a process for preparing a particle of cleavage type gag protein belonging to the family Retrovirus, a particle prepared by the process, and a vaccine comprising the particle as an effective ingredient. More particularly, the present invention relates to a process for preparing a particle of the cleaved gag protein which comprises culturing an animal culture cell infected with a virus vector free of env gene of a virus belonging to the family Retrovirus, in which at least gag gene and pol gene have been incorporated, or with a virus vector free of env gene of a virus belonging to the family Retrovirus, in which at least gag gene has been incorporated, or with a virus vector free of env gene of a virus belonging to the family Retrovirus, in which at least gag gene has been incorporated, and a virus vector free of env gene of a virus belonging to the family Retrovirus, in which at least pol gene has been incorporated; expressing, and cleaving the gag protein precursor; a particle thus prepared and a vaccine comprising the particle as an effective ingredient.

### [BACKGROUND OF THE INVENTION]

Recently, the onset and spread of acquired immunodeficiency syndrome (AIDS) have been seriously concerned on a worldwide level, and countermeasures for its prevention and treatment have become an urgent issue.

Since a few years ago, various retroviruses considered to be the causative viruses of AIDS or adult T cell leukemia have been isolated and identified, and nucleotide sequences of these genes have been determined. As the result, these viruses have been reported to be analogous or the same. In particular, HTLV-III, LAV and ARV which were considered to be the pathogenic viruses of AIDS are the same one and collectively named HIV (human immunodeficiency virus).

Retrovirus particles are composed of many proteins, which are named envelope glycoprotein (called env gene; in the case of HIV-I, env-gp 160 which is a precursor is cleaved to form two env proteins of gp120 and gp41), reverse transcriptase (in the case of HIV-I, there are p65 and p51), nucleoprotein (called gag protein; in the case of HIV-I, gag-p55 which is a precursor is cleaved to form 3 gag proteins of p17, p24 and p15); in the amino acid sequence of gag-p55, amino acid sequence corresponding to p17, amino acid sequence corresponding to p24 and amino acid sequence corresponding to p15 are continued in this succession), and the like. In addition, regulatory gene products such as tat, vif, rev, nef, etc. which are considered to be specific to HIV are known but other than vif product are not present in HIV particles.

gp120 which is env protein has the function of fixing viral particles to cell membrane upon infection, and gp41 has the function of allowing viral gene to invade into cells. p24 which is gag protein is the main component of core protein, and p17 is considered to be a matrix protein. Reverse transcriptase is coded by pol gene. pol gene additionally encodes p10 (protease protein), p31 (which is endonuclease and also reported to have an integrase activity); a group of these virus enzymes encoded by pol are called pol protein. vif encodes p23 (which is considered to take part in construction of virus particles).

Where an effective vaccine for AIDS is desired to obtain, it is necessary that an antigen suited to induce an antibody capable of neutralizing viruses be contained. As such an antigen, env protein, especially gp120 (Cell, 53, 483 (1988)) which is believed to be associated with infection ability is chosen as the most promising candidate. Studies on production of gp120 by genetic manipulation technique by incorporating a gene encoding this antigen protein into E. coli (e.g., Science, 228, 93-96 (1985)) have also been made.

However, analytical results of nucleotide sequence of HIV genome isolated from various patients or infected patients reveal that env gene of HIV mutates extremely vigorously (Scientific American, 253, 84-93 (1985)). It is thus unlikely that the technique utilizing this site would be effective for all HIV.

To the contrary, there is a possibility that a gene encoding gag protein which is the skeleton of virus particles or pol gene encoding reverse transcriptase, etc. would have a low probability of causing mutation. It is thus considered that vaccines comprising these genetic products as antigens would be advantageous. Therefore, studies have been made on production by genetic manipulation of integrating reverse transcriptase into E. coli (e.g., Science, 236, 305-308 (1987)), production of gag-p55 or p24 in the system of recombinant multinucleate virus (e.g., Virology, 158, 248-250 (1987)), synthesis of polypeptide which binds to an antibody capable of specifically binding to the antigenic determinant of p17 (Japanese Patent KOKAI (Laid-Open) No. 63-27498) and the like, including a report on the activity of a monoclonal antibody of p17 for neutralizing AIDS (J. Virol., 63, 267-272 (1989)). The present inventors also publicly announced a vaccinia virus integrated with gag gene and pol gene at the Meeting of the Association of AIDS Study in 1987, October.

Expression of non-cleavage type gag protein such as gag-p55, etc. was confirmed by recombination of gag gene as a whole in retrovirus but in this case, expression of cleavage type gag protein was not confirmed, irrespective of recombination of pol gene.

Furthermore, non-infectious retrovirus particles bearing gp160 and gp120 are known (WO88/09670). However, the retrovirus particles are obtained by treating retrovirus but it was unknown to prepare an env protein-free retrovirus core structure without forming retrovirus.

Gowda, S.D. et al. J. Virology 1989, Vol. 63, no. 3 pages 1451-1454 reports on a recombinant vaccinia virus which contains a 5.5 kb HIV gag-pol coding region. This recombinant was used to infect B-lymphoblastoid cells and evidence is disclosed indicating expression of core structure particles of a 24kDa protein. No disclosure concerning recovery of this protein is made.

### SUMMARY OF THE INVENTION

As a result of extensive studies, the present inventors have found that by expressing gag protein and pol protein in a cell infected with a virus vector without expressing env protein of retrovirus, cleavage type gag protein is expressed and a core structure particle of retrovirus having the same antigenicity as a part of the structure of retrovirus particle composed of the cleavage type gag protein and having no infectivity is obtained, and have come to accomplish the present invention.

### BRIEF DESCRIPTION OF THE DRAWINGS

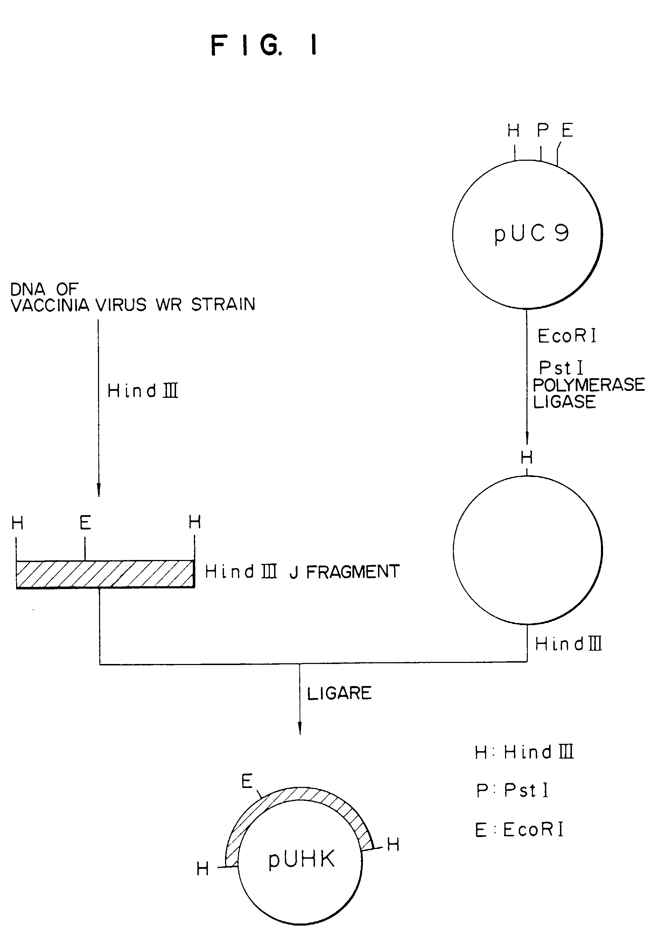

Fig. 1, Fig. 2 and Fig. 3 indicate the procedures of constructing plasmid pUHK, plasmid pNZ68K2 and plasmid W-gag, respectively.

### DETAILED DESCRIPTION OF PREFERRED EMBODIMENTS

In the present invention, the term "env protein" refers to env protein which governs infection or pathogenicity of retrovirus, and its mutants or variants, but does not mean modified env protein which has lost influence on infection or pathogenicity. In the present invention, the term "env gene-free" is used to mean that a gene encoding env protein is not contained in such a form that the gene can be expressed but may contain a gene derived from env gene irrelevant to infection or pathogenicity of retrovirus (a part of env gene or modified env gene, etc.). In gp160 and gp120-free retrovirus particles disclosed in WO88/09670, there is a less possibility that the included gene is taken up into the cell since gp120 is lacking; if the included gene were taken up into the cell, there is a danger that retrovirus might be constructed because of the whole gene of retrovirus; however, the particle of the present invention is free of env gene so that there is no such danger. Also in the present invention, however, where modified env gene is contained, it is possible to recover the pathogenicity by mutation or the like. Therefore, it is preferred that even modified env gene is not contained.

The retrovirus used in the present invention is not particularly limited so long as it can express gag protein and/or pol protein in an animal culture cell infected with a virus vector and is free of env gene. Examples include a virus belonging to the subfamily Oncovirus or the subfamily Rentivirus, specifically, HIV-I, HIV-II, HTLV-I and HTLV-II.

In the present invention, cDNA of retrovirus incorporated into a virus vector is not particularly limited as long as it can express gag protein and/or pol protein in an animal culture cell infected with a virus vector and is free of env gene. For example, env gene-free cDNA encoding gag gene and pol gene is obtained in the same manner as that for pNL43 (J. Virol., 59, 2, 284 (1986)), either by once cloning HIV-I incorporated into chromosome of the patient with AIDS in the form of provirus using λ phage and constructing a plasmid bearing the whole cDNA of HIV-I, or by subjecting RNA genome of HIV-I isolated from the patient to reverse transfer thereby to transform into cDNA, then transfer onto a plasmid, constructing the plasmid bearing the whole cDNA of HIV-I, digesting the resulting plasmid with an appropriate restriction enzyme and preparing a DNA fragment containing the region encoding both gag protein and pol gene.

The virus vector used in the present invention is not particularly limited as long as it can infect an animal cell and can express gag protein and pol gene. Examples include vaccinia virus, Avipox virus, Baculovirus, etc.

A method for construction of the virus vector incorporated with gag gene and/or pol gene which can be used in the present invention is not particularly limited, either. For example, where vaccinia virus is used as a virus vector, the desired virus vector can be constructed by the method disclosed in Japanese Patent KOKAI (Laid-Open) No. 1-74982, except that gag gene and/or pol gene of retrovirus is used as a gene to be incorporated instead of te gene encoding surface antigen protein of Japanese encephalitis virus and vaccinia virus used does not necessarily meet the preferred conditions.

Next, the thus constructed virus vector in which the cDNA fragment encoding gag gene and pol gene has been incorporated and the virus vector in which cDNA fragment encoding pol gene has been incorporated are infected to an animal culture cell, to express cleavage type gag protein and pol protein in the culture supernatant.

The animal culture cell used in the present invention may be the one where the virus vector can grow. Specific examples include, where the virus vector is vaccinia virus, TK-143 (derived from human osteosarcoma), FL (derived from human aminon), HeLa (derived from human cervical carcinoma), KB (derived from human pharyngeal cancer), RK13 (derived from rabbit kidney), CV-1 (derived from monkey kidney), BSC-1 (derived from monkey kidney), L929 (derived from mouse connective tissue), CE (chick embryo) cell, CEF (chick embryo fibroblast), SW480 (derived from human colon cancer), and the like. Where the virus vector is Avipox virus, chick derived cells such as chick embryo fibroblast, etc. are exemplified, also including grown egg chorioallantoic membrane. Where the virus vector is Baculovirus, examples include Sf9 strain derived from Spodoptera furugiperda and the like.

Under general culture conditions, however, an amount of cleavage type gag protein expressed is low, depending on the cell used; for example, where vaccinia virus obtained by incorporating with HIV-I gene in CV-1 is infected, cleavage type gag protein is hardly expressed. It is preferred to use the virus vector having a large expression amount of cleavage type gag protein in combination with an animal culture cell; an example of such combination includes SW480 infected with vaccinia virus incorporated with the gene of HIV-I.

Cleavage of gag protein expressed herein is considered to be caused by pol protein.

Purification of cleavage type gag protein from the culture supernatant is not particularly limited. For example, the culture supernatant is subjected to sucrose or potassium tartarate density gradient centrifugation in a conventional manner (Science, 224, 497-500 (1984)) followed by cesium chloride sedimentation equilibrium centrifugation. The cleavage type gag protein forms particles and the particles are recovered.

The particle composed of the cleavage type gag protein of the present invention is noted in the same fraction showing a specific gravity of about 1.15 as in HIV-I particles, where the retrovirus is HIV-I and fractionation is performed by the specific gravity.

A method for confirming that the particle is present in what fraction in each step of purification is not particularly limited. For example, there are a method using an antibody to gag protein and a method for measuring the activity of reverse transcriptase. The particle composed of the cleavage type gag protein of the particle may or may not include reverse transcriptase but in general, at least a part of it includes reverse transcriptase. Therefore, by measuring the activity of reverse transcriptase, the fraction containing the particle of the present invention can be identified.

The activity of reverse transcriptase may be measured in a conventional manner. For instance, uptake of ³²P-dTTP into the acid insoluble fraction in the culture supernatant infected with the recombinant virus into may be determined using poly-A as a template and oligo-dT as a primer, according to a modification of the description in "BISEIBUTUGAKU JISSHU TEIYO (Practice of Microbiology)" (edited by the Alumni Association of Tokyo University, Institute of Medical Science).

The particle of the present invention is accompanied by envelope-like thing which is considered to be lipid double membrane derived from vital membrane of the animal culture cell infected with the virus vector. The particle functions as an antigen even though it has the lipid double membrane, but where the presence of the lipid double membrane is not desired, the lipid double membrane may be destroyed and removed by treatment with an organic solvent such as ether under such conditions that the protein is not inactivated, or by freezing and thawing treatment.

The particle does not show pathogenicity, because it neither contains env protein which governs infection to the cell nor its gene. Other genes may or may not be contained. Likewise, the particle may or may not contain proteins other than env protein, so long as its structure is constructed with the cleavage type gag protein. Furthermore, the particle may or may not contain envelope-like lipid double membrane. The particle has an antigenicity and its antigenicity is derived from pol protein and gag protein, where gag protein and pol protein which are considered to cause less mutation than env protein are included. Furthermore, the particle has the same structure as that of a part of virus particles in the natural world, or the structure similar thereto and hence, the antigenicity of more potent than in gag protein, pol protein or a mere mixture thereof may be expected.

Retrovirus is confirmed or assumed to be a pathogenic virus of many fatal diseases such as AIDS, adult T cell leukemia, cancer, rheumatism, etc., and any vaccine for most of the diseases caused by retrovirus was unknown. Excellent effects can be expected from the particle of the present invention which as the same structure as the core of retrovirus or similar thereto.

According to the present invention, there is thus obtained the core structure of retrovirus free of env protein and env gene which is constructed by the cleavage type gag protein obtained by culturing a cell infected with the virus vector incorporated with cDNA encoding gag protein derived from retrovirus and cDNA encoding pol protein in a genome region non-essential for growth of the virus. The structure has the same as a part of the structure of retrovirus particle but has no infectivity. Therefore, it is expected that using the retrovirus core structure as the effective ingredient, effective retrovirus vaccine can be obtained.

Hereafter the present invention is described more specifically, with reference to the examples.

### Example 1

### (1) Preparation of recombinant vector pUHK (cf., Fig. 1)

After pUC9 (manufactured by Pharmacia) was digested with Eco RI and Pst I, the digestion product was treated with polymerase. The digested end was made blunt and ligated to prepare Eco RI site-deficient pUC9. After Eco RI site-deficient pUC9 was digested with Hind III, the digestion product was ligated with the Hind III J fragment of vaccinia virus WR strain bearing vaccinia virus TK gene to obtain recombinant vector pUHK.

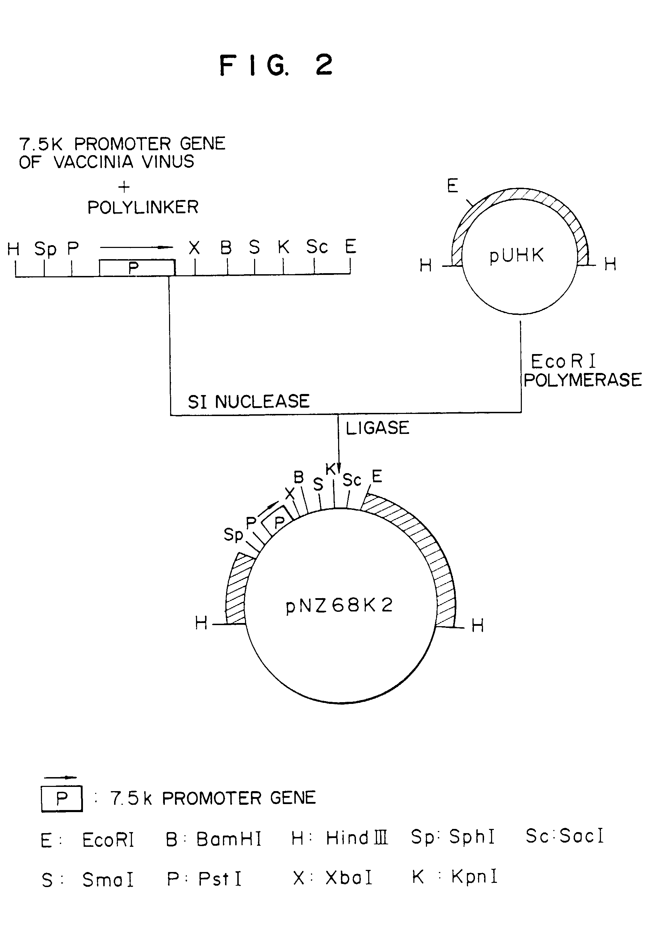

### (2) Preparation of recombinant vector pNZ68K2 (cf., Fig. 2)

After 7.5 K promoter of vaccinia virus WR strain (Moss et al., Cell, 125, 805-813 (1981)) was inserted into pUC19 (manufactured by Pharmacia) at the Hinc II site, the digestion product was cleaved successively with Hind II and Eco RI to obtain DNA fragment (about 350 bp) having the polylinker site upstream and downstream 7.5 K promoter.

The product obtained by treating this DNA fragment with Sl nuclease was ligated with the polymerase treatment product of the digestion product obtained by plasmid pUHK obtained in (1) with Eco RI. The thus obtained recombinant plasmid was named pNZ68K2.

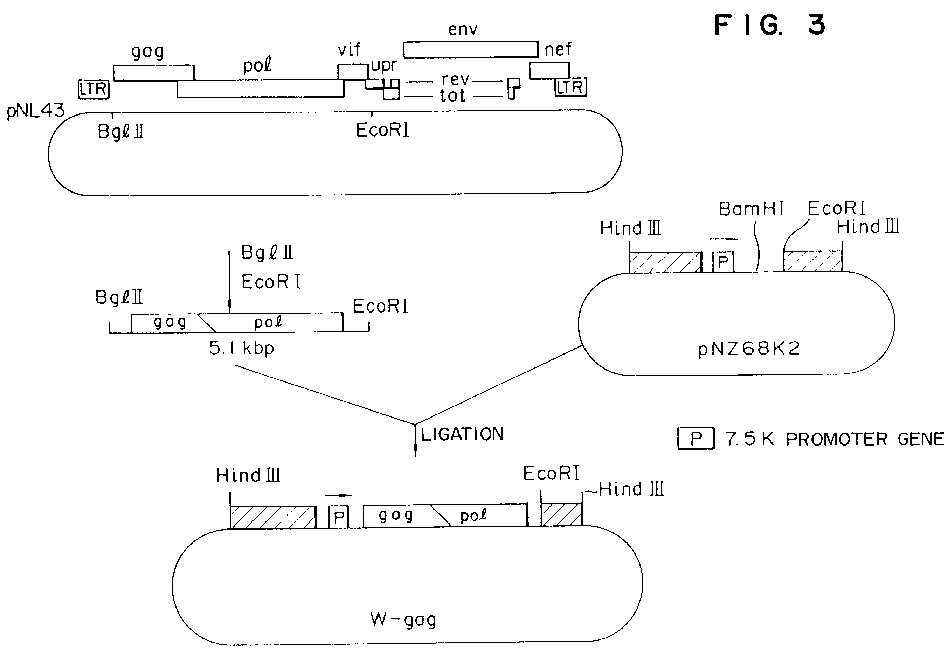

### (3) Preparation of recombinant plasmid W-gag (cf., Fig. 3)

After pNL43 containing the whole cDNA of HIV-I was digested with Bgℓ II and Eco RI, the fraction of about 5.1 kbp was isolated by low melting point agarose electrophoresis. The fraction was recovered by phenol extraction.

In the fraction, the region of gag gene and pol gene are contained, as shown in Fig. 3.

Next, pNZ68K2 obtained in (2) was digested with BamH I and Eco RI, the digestion product was ligated with the previously prepared DNA fragment containing gag gene and pol gene to obtain recombinant plasmid W-gag.

### (4) Production of recombinant vaccinia virus (cf., Fig. 3)

Vaccinia virus WR strain was inoculated on CV-l cell cultured in a culture bottle of 15 cm² in 1 p.f.u./cell. Fourty-five minutes later, the aforesaid recombinant plasmid W-gag was dissolved in 2.2 ml of sterilized water. DNA-calcium phosphate coprecipitates were prepared by the method of Hidaka et al. (PROTEIN, NUCLEIC ACID, ENZYME, 27, 340 (1985)) and 0.5 ml of the coprecipitates was dropped onto the infected CV-l cell. The system was allowed to stand at 37°C for 30 minutes in a 5% CO₂ incubator and 4.5 ml of Eagle's MEM medium containing 5% fetal calf serum. Three hours after, the culture broth was exchanged and the culture cell was frozen and thawed 3 times 48 hours after followed by ultrasonication for 30 seconds.

For confirmation of the inserted gag gene and pol gene into the recombinant, the aforesaid plaque-forming virus was inoculated on TK negative (TK⁻) 143 cell cultured in a Petri dish of 6 cm and 45 minutes after, Eagle's MEM medium supplemented with 1% agar, 12% fetal calf serum and 25 µg/ml BUdR was overlaid thereon. After culturing for 3 days, the infected cell was stained with 0.01% Neutral Red. The formed plaque was isolated with a Pasteur pipette and diluted in Eagle's MEM medium. With respect to the isolated plaque, the same procedure was again repeated to purify the plaque. The virus obtained from the purified plaque was proliferated and DNA was prepared from the proliferated virus. By dot blot hybridization using cDNA encoding gag gene and pol gene as a probe, the recombinant vaccinia virus was obtained. It was confirmed that the recombinant vaccinia virus was a recombinant virus bearing DNA encoding HIV-I-derived gag gene and pol gene into genome. The recombinant vaccinia virus was named V-20-1-8.

### (6) Expression of recombinant on the culture supernatant

V-20-1-8 was inoculated on SW480 cell cultured in a dish of 9 cm for tissue culture in m.o.i. of 10 to 20. After infecting at 37°C for an hour, the viral solution was withdrawn and the cell was washed with Eagle's MEM. Eagle's MEM supplemented with 5% fetal calf serum was added to the system. The culture supernatant was recovered 16 hours after and overlaid on a density gradient of 20-60% potassium tartarate prepared from 10 mM Tris-HCl (pH 7.4)-0.1 M NaCℓ - 1 mM EDTA solution. Centrifugation was performed at 35,000 rpm for 16 hours with Rotar SW47 (manufactured by Beckman). After centrifugation, the system was fractionated into each fraction.

To 5 ml of the reaction solution containing 50 mM Tris-HCℓ (pH 8.0), 10 mM dithiothreitol, 75 mM KCℓ, 5 mM MgCl₂, 10 µg/ml poly-A, 5 µg/ml oligo-dT, 0.05% NP40 was added 5 to 10 µl of [α-³²P]-dTTP (400 Ci/mmol, 10 mCi/ml). The reaction solution was separately charged in a 96-well U-shaped plate by the number of the fractions and control and, 10 ml of each fraction or 10 mM Tris-HCℓ (pH 7.4)-0.1 M NaCℓ-1 mM EDTA solution for control was added thereto. The reaction was conducted at 37°C for an hour. During the reaction, checkerboard scales with 2 cm spacing were written with a pencil on a nitrocellulose filter. After the reaction was completed, 10 µl of each fraction and the reaction solution for control were spotted onto each scale on the nitrocellulose filter. After the filter was completely air-dried, the filter was washed while shaking in 5% trichloroacetic acid-5% sodium pyrophosphate for 5 minutes, thereby to remove the unreacted radioactive precursor. Subsequently, the filter was washed in 5% trichloroacetic acid for 5 minutes and then in 50% ethanol for 5 minutes. After drying, each fraction was cut off along the checkerboard scales. Each fraction was put in a scintillation vial and the uptake was determined in a toluene scintillator by a liquid scintillation counter. The uptake showing more than 3 times than that of control was made positive and judged to have the reverse transcriptase activity.

Next, the fraction which was recognized with the reverse transcriptase activity was fractionated by SDS-polyacrylamide gel electrophoresis and blotted onto a nitrocellulose filter (Anal. Biochem., 112, 195-203 (1981)). Expression of the cleavage type gag protein was confirmed by so-called Western blotting utilizing ¹²⁵I-labeled protein A and anti-p24 monoclonal antibody (Japanese Journal of Cancer Research, 78, 235-241 (1987)) for detection. It was revealed that V-20-l-8-infected SW480 cell produced the cleavage type gag protein p24 having a molecular weight of 24,000 daltons in the supernatant. Judging from the structure of gap-p55, p24 corresponding to the amino acid sequence at the central portion of gag-p55 was produced; it is thus considered that p17 and p15 corresponding to the amino acid sequences of both ends of gag-p55 would also be produced.

The fraction having a specific gravity of about 1.15 from which the reverse transcriptase activity was detected was further purified by cesium chloride precipitation equilibrium centrifugation. The production of p24 was confirmed in the fraction having a specific gravity of about 1.15 from which the reverse transcriptase activity was detected.

By so-called negative staining, the aforesaid fraction in which p24 was confirmed was observed by an electron microscope, whereby spherical particles having a diameter of about 110 to about 150 nm different from vaccinia virus were noted.

### [INDUSTRIAL APPLICABILITY]

According to the present invention, the core structure of retrovirus constructed by the cleavage type gag protein can be prepared without being accompanied by env protein or env gene having infectivity or allowing to express infectivity. Therefore, it is highly likely that retrovirus vaccine with a minimized problem in view of safety would be provided. The present invention is thus highly applicable from an industrial viewpoint.

## Claims

1. A process for preparing a core structure particle of cleavage type gag protein comprising the steps of:
infecting a cell with a recombinant vaccinia virus containing the gag and pol genes from the same retrovirus or optionally, co-infecting a cell with two recombinant vaccinia viruses, one of said vaccinia viruses containing the gag gene and the other of said vaccinia viruses containing the pol gene from the same retrovirus, wherein none of said recombinant vaccinia viruses contain env gene sequences;
culturing the infected cell to express said core structure particle; and said process being characterized by
recovering said core structure particle from a culture supernatant fraction having a specific gravity of about 1.15 from which a reverse transcriptase activity is detected.

2. The process for preparing a core structure particle according to claim 1, wherein said retrovirus is HIV-I, HIV-II, HTLV-I OR HTLV-II.

3. The process for preparing a core structure particle according to claim 1, wherein said cell is a mammalian cell.

4. The process for preparing a core structure particle according to claim 3, wherein said mammalian cell is a SW480 cell derived from human colon cancer.

5. A core structure particle of cleavage type gag protein which has a particle size of 110 to 150nm prepared by the process according to claim 1.

6. An anti-retroviral vaccine comprising as an effective ingredient a core structure particle according to claim 5.

## Patentansprüche

1. Verfahren zur Herstellung eines Kernstrukturpartikels mit dem Spaltungstyp des gag Proteins, umfassend die Schritte:
Infizieren einer Zelle mit einem rekombinanten Vacciniavirus, enthaltend die gag- und pol-Gene vom gleichen Retrovirus oder gegebenenfalls Coinfizieren einer Zelle mit zwei rekombinanten Vacciniaviren, wobei eines der Vacciniaviren das gag-Gen und das andere das pol-Gen vom gleichen Retrovirus enthält, wobei keine der rekombinanten Vacciniaviren env-Gensequenzen enthält;
Züchten der infizierten Zelle zur Expression der Kernstrukturpartikel, wobei das Verfahren dadurch gekennzeichnet ist, daß das Kernstrukturpartikel aus einer Kulturüberstandsfraktion mit einem spezifischen Gewicht von etwa 1,15 und einer nachweisbaren Reverse-Transkriptaseaktivität gewonnen wird.

2. Verfahren zur Herstellung eines Kernstrukturpartikels nach Anspruch 1, wobei das Retrovirus HIV-I, HIV-II, HTLV-I oder HTLV-II ist.

3. Verfahren zur Herstellung eines Kernstrukturpartikels nach Anspruch 1, wobei die Zelle eine Säugerzelle ist.

4. Verfahren zur Herstellung eines Kernstrukturpartikels nach Anspruch 3, wobei die Säugerzelle eine SW480-Zelle, abgeleitet von einem menschlichen Dickdarmkrebs, ist.

5. Kernstrukturpartikel mit dem Spaltungstyp des gag-Proteins, hergestellt nach Anspruch 1, das eine Partikelgröße von 110 bis 150 nm hat.

6. Antiviraler Impfstoff, umfassend als Wirkstoff ein Kernstrukturpartikel nach Anspruch 5.

## Revendications

1. Procédé de production de particules de structure de noyau viral qui comportent une protéine *gag* clivée, lequel procédé comporte les étapes consistant :
à infecter une cellule avec un virus de vaccine recombiné, contenant les gènes *gag* et *pol* d'un même rétrovirus, ou bien, le cas échéant, co-infecter une cellule avec deux virus de vaccine recombinés dont l'un contient le gène *gag* et l'autre le gène *pol* d'un même rétrovirus, mais aucun de ces virus de vaccine recombinés ne contenant la séquence du gène *env,*
et à cultiver la cellule infectée pour qu'elle exprime ladite particule de structure de noyau viral,
ledit procédé étant caractérisé en ce que l'on récupère ladite particule de structure de noyau viral dans une fraction du surnageant de culture dont la densité vaut environ 1,15 et où l'on a détecté une certaine activité de transcriptase inverse.

2. Procédé de production de particules de structure de noyau viral, conforme à la revendication 1, dans lequel ledit rétrovirus est VIH-I, VIH-II, HTLV-I ou HTLV-II.

3. Procédé de production de particules de structure de noyau viral, conforme à la revendication 1, dans lequel ladite cellule est une cellule de mammifère.

4. Procédé de production de particules de structure de noyau viral, conforme à la revendication 1, dans lequel ladite cellule de mammifère est une cellule SW480 dérivée d'une tumeur cancéreuse de côlon humain.

5. Particule de structure de noyau viral, comportant une protéine *gag* clivée, dont la taille vaut de 110 à 150 nm et qu'on a préparée selon un procédé conforme à la revendication 1.

6. Vaccin anti-rétroviral comportant, comme ingrédient actif, une particule de structure de noyau viral conforme à la revendication 5.
